# EUROPEAN PATENT SPECIFICATION

(11) **EP 1 474 189 B1**
(45) Date of publication and mention of the grant of the patent: **06.04.2011**
(21) Application number: 02709447.3
(22) Date of filing: 11.02.2002
(51) Int. Cl.: A61M 1/06

(54) **LUBRICATED BREASTSHIELD**
GESCHMIERTE BRUSTHAUBE
PROTÈGE-SEIN LUBRIFIÉ

(43) Date of publication of application: 10.11.2004
(60) Divisional application: 10009311.1
(73) Proprietor: Medela Holding AG, 6340 Baar (CH)
(72) Inventor: SILVER, Brian, H., Cary, IL 60013 (US)
(74) Representative: Clerc, Natalia
(86) International application number: PCT/US2002/003896
(87) International publication number: WO 2003/068291

(56) References cited:
- EP-A- 1 034 807
- EP-A2- 0 385 933
- US-A- 2 542 505
- US-A- 4 799 922
- US-A- 5 032 113
- US-A- 5 308 321
- US-A- 5 885 246
- US-B1- 6 290 671

## Description

### FIELD OF THE INVENTION

The invention relates to breastshields used with breastpumps by nursing mothers.

### BACKGROUND OF THE INVENTION

Breastshields typically used in conjunction with breastpumps have a conical configuration and are usually funnel-shaped. The breastshield need not be conical, of course, but nonetheless will ordinarily have a part within which at least some of the breast is received along with the nipple. In use, the interior of the breastshield is placed against the nursing mother's breast, and the breastpump extracts milk from the breast through application of a negative pressure (vacuum) within the breastshield. Breastpumps having breastshields of this type are shown and described in U. S. Patent Nos. 4,929, 229 and 4,857, 051, for instance.

US 6 290 671 discloses a breast pump with a breast shield, the pump being integrated on a body of a regulating device. The regulating device can be made of polytetrafluoroethylene (PTFE).

EP 1 034 807 shows a breast shield with a liner wherein the conical portion of the liner is provided with a structured or corrugated surface. The liner is made of a soft, resilient elastomer, such as a silicone rubber.

US 5 308 321 shows a breast shield used for medical purposes. Before use, a lubricant can be applied to the breast shield so as to improve the pressure seal between the cup member and the breast portion.

Breastshields typically have a funnel-shape, comprising a conical portion with a tubular extension, sometimes referred to as the nipple tunnel. The nipple and surrounding breast are received in the conical portion, with the nipple often extending into the tubular extension. Under vacuum, the breast is pulled further into the breastshield, ordinarily with the nipple then being pulled into the tubular extension, with the surrounding breast thereby also compressed about the nipple.

A nursing mother's nipples may thus be forced against the wall of the nipple tunnel under vacuum. This may cause friction against the wall as the nipple moves deeper into the nipple tunnel. There can also be friction between the breast and the conical portion, as well as the nipple tunnel. This may result in irritation, particularly if the mother is already suffering from some topical problem, such as a chapped or cracked nipple condition. This makes the pumping of milk from the breast uncomfortable. If it were possible to reduce or eliminate the chafing and irritation of a nursing mother's breast when a breastpump is used, an advance in the art would be afforded.

### SUMMARY OF THE INVENTION

The invention comprises a breastshield and a breastpump with the features of claims 1 and 7 respectively. The breastshield for a breastpump has a breast-receiving portion which is provided with a lubricious surface. In one embodiment, it has bonded to its interior breastcontacting surface a coating of a polymeric lubricant, having a reduced coefficient of friction. The polymeric lubricant may preferably be from the group consisting of polytetrafluoroethylene, fluorinated ethylene-propylene copolymers, poly (vinylidine fluoride), polyparaxylene and silicone lubricant polymers. Preferred polymers of this group are the polyparaxylene polymers and the polytetrafluoroethylene polymers, with parylene being a presently preferred lubricating material. It is to be understood that any number of other well-known lubricating materials may be used in the practice of the invention, as will be made evident hereafter. Due to the particular application of the invention as a breast shield, it is necessary that the particular polymers chosen be acceptable for bodily contact.

While the lubricating material may be bonded directly to a rigid breast-receiving part of the breastshield, which could also be an insert or adaptor received in a breastshield, it is furthermore envisioned to provide the lubricious surface in conjunction with elastomeric materials making up a soft breast-receiving part. Whether a rigid or softer substrate is employed, the lubricating material can be provided as a coating to the already formed substrate (or base). Alternatively, the two layers could be molded together in the same molding operation, or in a two-shot type molding process, just to name two other applications.

In one embodiment, the lubricating material is present on, provided to or applied to a major part of the breast-receiving surface of the breastshield. In an alternate embodiment, the lubricating material is provided to the entire breast-receiving surface of the breastshield.

In another embodiment, the lubricant polymer material is mixed with or otherwise contained within the substrate, to provide a relatively homogeneous lubricant-containing breastshield surface area.

Yet another variation of the foregoing embodiment has the lubricant material releasable upon contact with water, or heat. The lubricant may be encapsulated, for instance, and then released to coat the surface in use.

The foregoing objectives and advantages of the invention will be further understood upon consideration of the following detailed description of certain embodiments taken in conjunction with the drawings, in which:

### BRIEF DESCRIPTION OF THE DRAWINGS

Fig. 1 is a funnel-shaped breastshield having an interior lubricated surface; and
Figs. 2a and 2b are two embodiments of the invention each taken across the lines 2-2 of Fig. 1.

In the drawings, like parts have like numbers.

### DETAILED DESCRIPTION OF EMBODIMENTS OF THE INVENTION

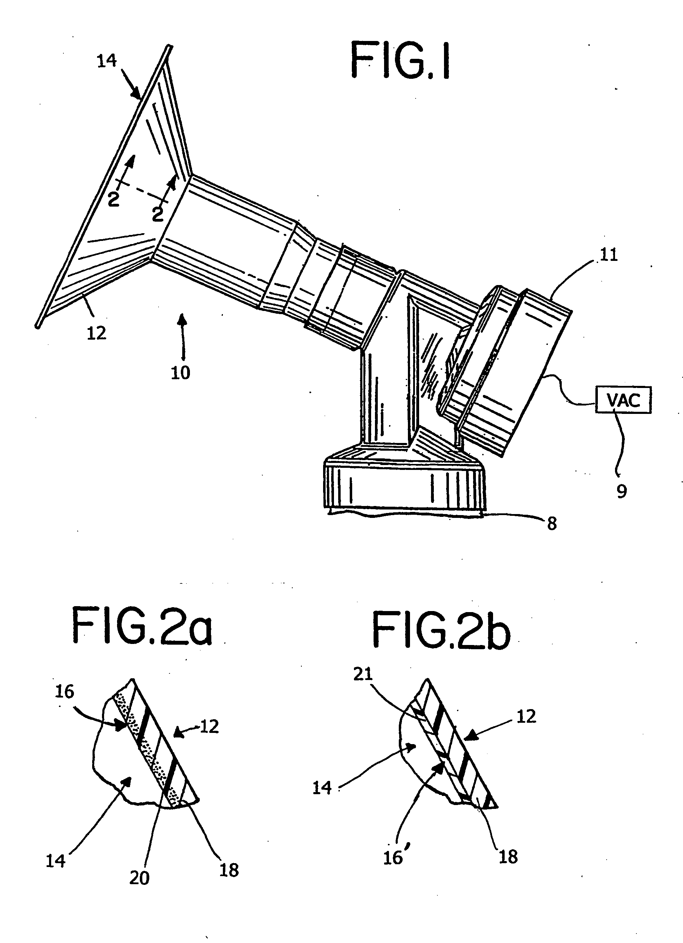

With respect to the drawings, there is shown in Fig. 1 a funnel-shaped breastshield 10 having a breast-receiving cone 12, the interior of which is designated by the numeral 14. This is a relatively well-known type of breastshield, having a rigid plastic construction. It will be understood, however, that the application of this invention is not limited to just such a form of the breastshield.

The breastpump assembly which includes the breastshield 10 further has a container 8 for collecting milk, and source of pressure, typically a vacuum (negative pressure), schematically indicated at 9. The vacuum source 9 would typically be a manually operated piston pump attached at collar 11, a battery operated diaphragm pump attached at collar 11, a house-current driven vacuum pump using a vacuum line (tube) attached to a port within the collar 11, among others. Again, reference can be made to U.S. 4,929,229 and 4,857,051 for such detail. The invention herein is, however, not limited to the manner in which the breast is manipulated for the expression of milk.

Figs. 2a and 2b show a lubricating area formed integral with the interior 14 of the main breast-receiving portion of cone 12. As shown in Fig. 2a, the area 16 is part of a base polymer 18 forming the general structure of the cone 12. Distributed about its interior surface and partially dispersed therein is a lubricant polymer in the form of intermingling particles 20, which are encapsulated for release in use.

Fig 2b shows a different embodiment from 2a, wherein a lubricious coating 16' is provided in the form of a discrete layer 21, such as one yielded through coating base polymer 18 with the lubricating material. This can be any number of lubricants, including but not limited to a dry lubricant material which reduces friction on contact with liquid.

The layer 21, in the form of a film, may be bonded by any suitable means. One particular lubricious material considered advantageous is parylene. A coating thickness in the range from about 1500 to about 2500 Angstroms may suffice. One type of parylene considered suitable is provided under the name ParyLAST, by AST Products, Inc. of Billerica, Massachusetts (see U.S. 5,355,382 and 5,447,799), and can be applied to a silicone substrate. Another lubricating material considered applicable is a coating material sold under the name HYDROMER by a company of the same name, and is a polyvinyl pyrrolidone interacted with an isocyanate prepolymer.

As indicated, it is possible to employ a single lubricating polymeric material to accomplish the goals of the invention. To effectuate the desired lower coefficients of friction, it is also possible to blend a variety of polymers, such as silicone polymers with fluorinated polymers with the lubricating material.

An elastomeric polymer considered useful as the substrate is KRATON G2705, a thermoplastic elastomer that is a block copolymer, having a synthetic rubber base and terminal polystyrene moieties. This polymer has been approved for use in medical devices. This product is available from GLS Corporation of Cary, Illinois. It typically has the following physical properties:

| | |
|---|---|
| Hardness, Shore A, (ASTM D2240) injection molded | 57 |
| Specific Gravity, (D792) | 0.90 |
| Tensile Modulus at 300% Elongation, PSI, (D412) In Flow Direction | 405 |
| Tensile Strength at Break, PSI, (D412) In Flow Direction | 970 |
| Percent Elongation at Break | 655 |
| Tear Strength, PLI, Die C, (D624) In Flow Direction | 136 |
| Color | Translucent |

It should be noted that this product by itself has little lubricity. Another useful lubricant polymer additive that could be applied to a Kraton G2705 base is a proprietary material sold under the trade name LUBRICOAT, by Coating Technologies Inc. of Scotch Plaines, New Jersey. This material has an exceptionally low coefficient of friction.

Yet another lubricant that has been considered is sold by Witco Corporation of Greenwich, Connecticut under the name KENAMIDE E ULTRA, and is an unsaturated fatty monamide derived from erucic acid. The material may be blended with the plastic making up the surface layer of the breastshield, for one example. KENAMIDE E ULTRA has the following specifications:

| | |
|---|---|
| Amide Content, % | 98.0 min. |
| Erucic Content, % by GLC | 90.0 min. |
| Color, Gardner (1963) | 2 max. |
| Acid Value | 1 max. |
| Iodine Value | 71-76 |
| Melting Point, °C (°F) | 76-86 (168-187) |
| Moisture, % | 0.25 max. |

Thus, while the invention has been described with respect to certain presently preferred embodiments, those with skill in the art will recognize other applications which will fall within the scope of the claims.

## Claims

1. A breastshield (10) for a breastpump having a breast-receiving part (12) within which a portion of a mother's breast is received including the nipple, **characterized in that** the breastshield comprises a lubricious surface layer (16, 21) molded together with or bonded to a major portion of the breast-receiving part (12).

2. The breastshield of Claim 1, wherein the lubricious surface layer (16, 21) is a polymeric material from the group consisting of polytetrafluoroethylene, fluorinated ethylene-probylene copolymers, poly (vinylidine fluoride), polyparaxylene and silicone lubricant polymers.

3. The breastshield of Claim 1, wherein the lubricious surface layer (16, 21) is parylene.

4. The breastshield of Claim 1, wherein the lubricious surface layer (16, 21) is intermingled with a non-lubricating base layer.

5. The breastshield of Claim 2, wherein the polymeric material is coated on the surface of a base layer (18) forming the structure of the breast-receiving part (12).

6. The breastshield of Claim 1, wherein a lubricant material is dispersed within a substrate and encapsulated therein for release in use of the breastshield.

7. A breastpump comprising a breastshield (10) having an interior portion (14) adapted to receive at least part of a mother's breast including the nipple therein;
a container (8) communicating with said breastshield (10) to receive milk; and
a pressure source (9) communicating with said interior portion (14) to manipulate the breast therein for the expression of milk;
**characterized in that** the breastshield comprises a lubricious surface layer (16, 21) molded together with or bonded to a major portion of said interior portion (14).

8. The breastpump of Claim 7, wherein said lubricious surface layer is a lubricant coating forming an outer layer to said interior portion.

9. The breastpump of Claim 7, wherein said lubricious surface layer is a lubricant material which is intermingled with a non-lubricating base layer.

10. The breastpump of Claim 9, wherein said lubricant material is dispersed within a substrate and encapsulated therein for release in use of the breasthield.

11. The improved breastshield of Claim 1, wherein the entire breast-receiving portion includes the lubricious surface layer bonded to the major portion of the breast-receiving part.

## Patentansprüche

1. Brusthaube (10) für eine Brustpumpe, aufweisend einen Brust-aufnehmenden Teil (12), innerhalb welchem ein Teil der Brust einer Mutter inklusive der Brustwarze aufgenommen ist, **dadurch gekennzeichnet, dass** die Brusthaube eine schmierende Oberflächenschicht (16, 21) aufweist, welche zusammen mit einem Hauptteil des Brust-aufnehmenden Teils (12) ausgeformt ist oder an einem Hauptteil des Brust-aufnehmenden Teils (12) haftet.

2. Brusthaube gemäss Anspruch 1, wobei die schmierende Oberflächenschicht (16, 21) ein polymerisches Material ist aus der Gruppe bestehend aus Polytetrafluorethylen, Fluorethylenpropylen, Poly (Vinylidenfluorid), Poly-Paraxylol und schmierenden Silikon-Polymeren.

3. Brusthaube gemäss Anspruch 1, wobei die schmierende Oberflächenschicht (16, 21) Parylene ist.

4. Brusthaube gemäss Anspruch 1, wobei die schmierende Oberflächenschicht (16, 21) mit einer nicht-schmierenden Basisschicht durchmischt ist.

5. Brusthaube gemäss Anspruch 2, wobei das polymerische Material auf die Oberfläche einer Basisschicht (18) aufgeschichtet ist, welche die Struktur des Brust-aufnehmenden Teils (12) bildet.

6. Brusthaube gemäss Anspruch 1, wobei ein schmierendes Material in einem Substrat dispergiert und darin eingekapselt ist, um bei Gebrauch der Brusthaube freigesetzt zu werden.

7. Brustpumpe aufweisend eine Brusthaube (10), welche einen inneren Anteil (14) hat, der dazu ausgebildet ist, zumindest einen Teil der Brust einer Mutter inklusive der Brustwarze aufzunehmen;
einen Behälter (8), welcher mit der Brusthaube (10) kommuniziert, um Milch aufzunehmen; und
eine Druckquelle (9), welche mit diesem inneren Anteil (14) kommuniziert, um darin die Brust zur Extraktion von Milch anzuregen;
**dadurch gekennzeichnet, dass** die Brusthaube eine schmierende Oberflächenschicht (16, 21) aufweist, welche zusammen mit einem Hauptteil des inneren Anteils (14) ausgeformt ist oder an einem Hauptteil des inneren Anteils (14) haftet.

8. Brustpumpe gemäss Anspruch 7, wobei diese schmierende Oberflächenschicht eine Schmiermittel-Beschichtung ist, welche eine äussere Schicht an diesem inneren Anteil bildet.

9. Brustpumpe gemäss Anspruch 7, wobei die schmierende Oberflächenschicht ein schmierendes Material ist, welches mit einer nicht-schmierenden Basisschicht durchmischt ist.

10. Brustpumpe gemäss Anspruch 9, wobei das schmierende Material in einem Substrat dispergiert und darin eingekapselt ist, um bei Gebrauch der Brusthaube freigesetzt zu werden.

11. Verbesserte Brusthaube gemäss Anspruch 1, wobei der gesamte Brustaufnehmende Anteil die schmierende Oberflächenschicht beinhaltet, welche am Hauptteil des Brust-aufnehmenden Teils haftet.

## Revendications

1. Téterelle (10) pour un tire-lait ayant une partie pour recevoir le sein (12) dans laquelle est reçue une portion du sein d'une mère incluant le mamelon, **caractérisée en ce que** la téterelle comprend une couche de surface lubrifiée (16, 21) moulée conjointement avec ou collée à une portion majeure de la partie pour recevoir le sein (12).

2. Téterelle selon la revendication 1, dans laquelle la couche de surface lubrifiée (16, 21) est un matériau polymère appartenant au groupe constitué du polytétrafluoroéthylène, des copolymères d'éthylène-propylène fluorés, de fluorure de polyvinylidène, de polyparaxylène et de polymères de silicone lubrifiants.

3. Téterelle selon la revendication 1, dans laquelle la couche de surface lubrifiée (16, 21) est en parylène.

4. Téterelle selon la revendication 1, dans laquelle la couche de surface lubrifiée (16, 21) est entremêlée à une couche de base non lubrifiante.

5. Téterelle selon la revendication 2, dans laquelle le matériau polymère est revêtu sur la surface d'une couche de base (18) formant la structure de la partie pour recevoir le sein (12).

6. Téterelle selon la revendication 1, dans laquelle un matériau lubrifiant est dispersé dans un substrat et encapsulé dans celui-ci en vue d'être libéré pendant l'utilisation de la téterelle.

7. Tire-lait comprenant une téterelle (10) ayant une portion intérieure (14) prévue pour recevoir au moins une partie du sein d'une mère incluant le mamelon ;
un récipient (8) communiquant avec ladite téterelle (10) pour recevoir le lait ; et
une source de pression (9) communiquant avec ladite portion intérieure (14) pour manipuler le sein s'y trouvant en vue d'exprimer du lait ;
**caractérisé en ce que** la téterelle comprend une couche de surface lubrifiée (16, 21) moulée conjointement avec ou collée à une portion majeure de ladite portion intérieure (14).

8. Tire-lait selon la revendication 7, dans lequel ladite couche de surface lubrifiée est un revêtement lubrifiant formant une couche extérieure de ladite portion intérieure.

9. Tire-lait selon la revendication 7, dans lequel ladite couche de surface lubrifiée est un matériau lubrifiant qui est entremêlé à une couche de base non lubrifiante.

10. Tire-lait selon la revendication 9, dans lequel ledit matériau lubrifiant est dispersé dans un substrat et encapsulé dans celui-ci en vue d'être libéré pendant l'utilisation de la téterelle.

11. Téterelle améliorée selon la revendication 1, dans laquelle la totalité de la portion pour recevoir le sein comporte la couche de surface lubrifiée collée à la portion majeure de la partie pour recevoir le sein.
